# EUROPEAN PATENT APPLICATION

(11) **EP 3 449 951 A2**
(43) Date of publication of application: **06.03.2019**
(21) Application number: 18190964.9
(22) Date of filing: 27.08.2018
(51) Int. Cl.: A61L 9/12, B60H 3/00, H04W 4/48, H04W 4/80

(54) **VEHICLE FRAGRANCE DISPENSER**

(30) Priority: 01.09.2017 JP 2017168886
(71) Applicant: TOYOTA JIDOSHA KABUSHIKI KAISHA, Toyota-shi, Aichi-ken, 471-8571 (JP)
(72) Inventor: YOSHIMATSU, Junichi, TOYOTA-SHI, AICHI-KEN, 471-8571 (JP)
(74) Representative: Cabinet Beau de Loménie

(57) **Abstract**

A vehicle fragrance dispenser (100) includes: a fragrance generation section (22) configured to perfume air within a vehicle (10) using one of a plurality of different perfumes; a transceiver section (60) configured to communicate with a mobile data terminal (70, 72) of an occupant; and a controller (20) configured to associate and register any one out of the plurality of perfumes with individual identification information of the mobile data terminal (70, 72) of the occupant, and, in a case in which the transceiver section (60) has received individual identification information of the mobile data terminal (70, 72) of the occupant, the controller (20) controls the fragrance generation section (22) to perfume the air within the vehicle (10) using a perfume associated with the received individual identification information.

## Description

### BACKGROUND

### Technical Field

The present disclosure relates to a fragrance dispenser for a vehicle.

### Related Art

Pleasant fragrances are released inside vehicles to improve the mood of occupants, or to make unpleasant odors in the vehicle less obvious (masking). Hitherto, fragrances have been dispersed inside vehicles using commercially available air fresheners. However, it takes time for such commercially available air fresheners to disperse sufficient fragrance inside vehicles.

Japanese Patent Application Laid-Open (JP-A) No. 2004-268704 discloses an invention relating to a vehicle fragrance generating device including a blower to create an airflow to carry fragrance generated by a fragrance generator.

However, the vehicle fragrance generating device disclosed in JP-A No. 2004-268704 is not capable of switching the type of fragrance to be released inside the vehicle according to the occupant of the vehicle.

### SUMMARY

The present disclosure provides a vehicle fragrance dispenser that may identify an occupant and releasing a fragrance associated with the occupant.

A first aspect of the present disclosure is a vehicle fragrance dispenser including: a fragrance generation section configured to perfume air within a vehicle using one of plural different perfumes; a transceiver section configured to communicate with a mobile data terminal of an occupant; and a controller configured to associate and register any one out of the plural perfumes with individual identification information of the mobile data terminal of the occupant, and, in a case in which the transceiver section has received individual identification information of the mobile data terminal of the occupant, the controller controls the fragrance generation section to perfume the air within the vehicle using a perfume associated with the received individual identification information.

In the vehicle fragrance dispenser of the first aspect, the occupant is identified by the individual identification information of the mobile data terminal carried by the occupant. Thus, the first aspect of the present disclosure may release a fragrance associated with the identified occupant.

In a second aspect of the present disclosure, in the above first aspect, in a case in which the transceiver section has received plural individual identification information associated with the perfumes, the controller may transmit a message to mobile data terminals matching the received individual identification information, and may control the fragrance generation section to perfume the air within the vehicle using a perfume indicated by a response to the message.

In the vehicle fragrance dispenser of the second aspect, in cases in which plural occupant mobile data terminals have been detected, a message is transmitted to the detected mobile data terminals. Thus, the second aspect of the present disclosure may release a fragrance according to a response to the message.

In a third aspect of the present disclosure, in the above aspects, the fragrance generation section may be configured to release a deodorizing component inside the vehicle to deactivate a fragrance component remaining inside the vehicle; and the controller may control the fragrance generation section to release the deodorizing component inside the vehicle in a case in which the occupant has left the vehicle has been detected.

The vehicle fragrance dispenser of the third aspect may effectively disperse fragrance of a different perfume by, after the occupant has left the vehicle, releasing a deodorizing component inside the vehicle to deactivate fragrance component remaining inside the vehicle.

In a fourth aspect of the present disclosure, in the above aspects, the fragrance generation section may be configured to release a tear gas component inside the vehicle; and the controller may control the fragrance generation section to release the tear gas component inside the vehicle in a case in which an illegitimate engine start has been detected by an immobilizer of the vehicle.

The vehicle fragrance dispenser of the fourth aspect may prevent theft of a vehicle by releasing a tear gas component inside the vehicle when there is an illegitimate engine start attempt in the vehicle.

### BRIEF DESCRIPTION OF THE DRAWINGS

Exemplary embodiments will be described in detail based on the following figures, wherein:
Fig. 1 is a block diagram illustrating configuration of a vehicle fragrance dispenser according to a first exemplary embodiment of the present disclosure;
Fig. 2 is a schematic diagram illustrating a fragrance generator according to the first exemplary embodiment of the present disclosure;
Fig. 3 is a flowchart illustrating processing of a vehicle fragrance dispenser according to the first exemplary embodiment of the present disclosure;
Fig. 4 is a schematic diagram illustrating a fragrance generator according to a second exemplary embodiment of the present disclosure; and
Fig. 5 is a flowchart illustrating deodorization processing of a vehicle fragrance dispenser according to the second exemplary embodiment of the present disclosure.

### DETAILED DESCRIPTION

### [First Exemplary Embodiment]

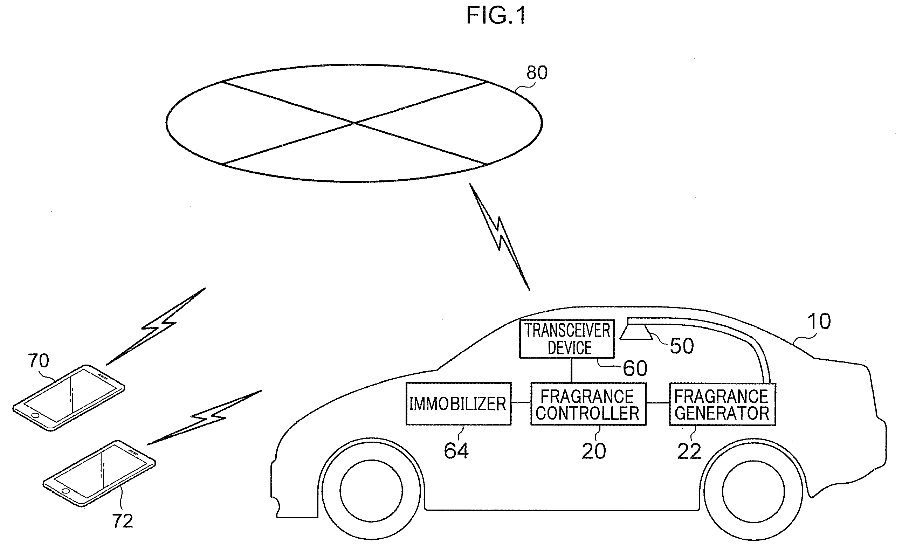

Explanation follows regarding a vehicle fragrance dispenser 100 according to an exemplary embodiment, with reference to Fig. 1 to Fig. 3. Fig. 1 is a block diagram illustrating an example of configuration of the vehicle fragrance dispenser 100 according to the present exemplary embodiment. The vehicle fragrance dispenser 100 illustrated in Fig. 1 includes a fragrance generator 22, a fragrance controller 20, a transceiver device 60, and an immobilizer 64. The fragrance generator 22 releases a fragrance component into the interior of a vehicle 10 through a blower vent 50. The fragrance controller 20 controls the fragrance generator 22. The transceiver device 60 is configured to enable communication with mobile data terminals 70, 72, such as smartphones, held by occupants, i.e. users. The immobilizer 64 prevents theft of the vehicle 10.

The transceiver device 60 directly receives signals that the mobile data terminals 70, 72 are constantly transmitting in order to search for the nearest base station, and is capable of two-way communication with the mobile data terminals 70, 72 over an existing network 80 configured by public communication lines, the internet, or the like.

The immobilizer 64 electronically compares a unique identification code of a transponder, which is an electronic chip embedded in a dedicated key, with an identification code of the vehicle 10, and starts the engine in a case in which the codes match. Theft of the vehicle 10 may be prevented by not starting the engine of the vehicle 10 if the codes do not match, or if there is no configuration corresponding to a transponder at all in the key employed.

The fragrance controller 20 extracts individual identification information included in the signal that the mobile data terminals 70, 72 are constantly transmitting to search for the nearest base station, as received by the transceiver device 60. The fragrance controller 20 then determines whether or not the extracted individual identification information matches individual identification information for a pre-stored mobile data terminal of an occupant. If the individual identification information included in the signal received by the transceiver device 60 matches the individual identification information of a pre-stored mobile data terminal of an occupant, the fragrance controller 20 controls the fragrance generator 22 such that a fragrance component registered in association with the individual identification information is released into the vehicle.

In cases in which the fragrance controller 20 receives plural pre-stored individual identification information signals, the fragrance controller 20 transmits a message to the mobile data terminals 70, 72 matching the received individual identification information. As an example, the message may take the form of an E-mail sent over the network 80. The E-mail prompts selection of the perfume to be employed, and therefore the user follows the text of the E-mail to send a reply (response) selecting the perfume to be employed. The fragrance controller 20 controls the fragrance generator 22 so as to select the perfume indicated by the response to the message.

In cases in which the immobilizer 64 has refused to start the engine, the fragrance controller 20 controls the fragrance generator 22 to release a component having a foul odor, such as tear gas, through the blower vent 50 in order to repel the person attempting to start the engine.

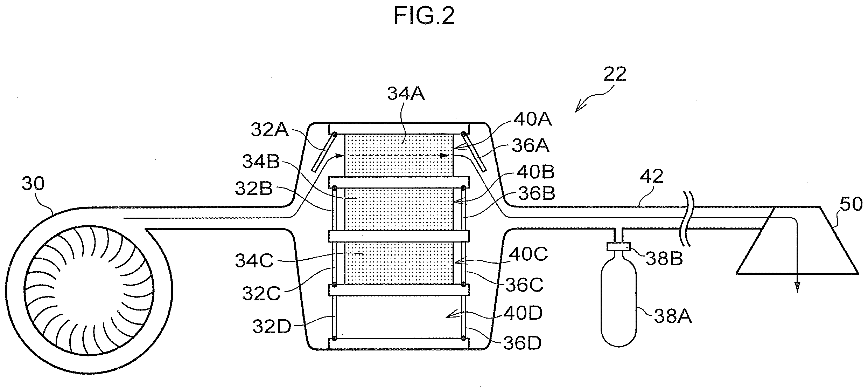

Fig. 2 is a schematic diagram illustrating an example of the fragrance generator 22 according to the present exemplary embodiment. The fragrance generator 22 illustrated in Fig. 2 includes cartridges filled with various different perfumes, such as a cartridge 34A, a cartridge 34B, and a cartridge 34C. A single cartridge is selected for use. For example, the cartridge 34A is filled with a mint-based perfume imparting a cooling sensation, the cartridge 34B is filled with a vanilla-based perfume that creates a warm, sweet feeling, and the cartridge 34C is filled with an essential oil-based perfume derived from a flower having a pleasant floral smell. Moreover, as well as mint-based perfumes, vanilla-based perfumes, and essential oil-based perfumes, cartridges may, for example, be filled with citrus-based perfumes or the like.

In the cartridges 34A, 34B, 34C, for example, a porous material that is well-suited to adsorbing the perfume and is a chemically inert substrate is charged with the perfume. Examples of substrates that may be employed include silica gel, alumina, and diatomaceous earths.

The cartridges 34A, 34B, 34C are loaded into respective channels 40A, 40B, 40C, these being airflow paths from a blower 30 configured by a multi-bladed fan rotated by an electric motor, such as a sirocco fan. As illustrated in Fig. 2, only a channel 40D is not loaded with a cartridge.

The channel 40A is provided with valves 32A, 36A. The channel 40B is provided with valves 32B, 36B. The channel 40C is provided with valves 32C, 36C. The channel 40D is provided with valves 32D, 36D. The valve 36A opens and closes in coordination with the valve 32A so as to open or close the channel 40A airflow path. Similarly, the valve 36B, the valve 36C, and the valve 36D open and close in coordination with the valve 32B, the valve 32C, and the valve 32D, respectively, so as to open or close the respective channels 40B, 40C, 40D. Each of the valves 32A to 36D is, for example, opened and closed by an electrical actuator.

Since each of the cartridges 34A, 34B, 34C is filled with a different perfume, the valves that are opened are any pair out of a pair configured by the valves 32A, 36A, a pair configured by the valves 32B, 36B, and a pair configured by the valves 32C, 36C. The valves 32D, 36D are closed while any valve pair, out of the valve 32A, 36A pair, the valve 32B, 36B pair, or the valve 32C, 36C pair, is open.

Moreover, when there is no need to release any of the perfumes filling the cartridges 34A, 34B, or 34C into the vehicle, the perfumes filling the cartridges 34A, 34B, 34C are prevented from being dispersed unnecessarily by closing all the valves 32A to 36C.

The valves 32D, 36D of the channel 40D not loaded with a cartridge are opened in order to release tear gas, filled in a tear gas cartridge 38A, into the vehicle through the blower vent 50. When the valves 32D, 36D are open in a state in which the valves 32A, 36A, 32B, 36B, 32C, 36C are all closed, the airflow from the blower 30 reaches an airflow path 42 via the channel 40D. By opening a solenoid 38B in this state, a tear gas component in the tear gas cartridge 38A is sprayed into the airflow path 42, and, together with the airflow, is released inside the vehicle through the channel 40D.

The tear gas cartridge 38A is a spray can type of container filled with the tear gas component in a liquid state together with a pressurized gas propellant. When the solenoid 38B is open, the tear gas component is sprayed into the airflow path 42 by the pressure of the pressurized gas.

Various tear gas components may be considered, and as an example, chloroacetophenone or the like may be employed, which is known to have relatively few side-effects on humans. Since chloroacetophenone is solid at room temperature, it is filled into the tear gas cartridge 38A in a state dissolved in a solvent such as ethanol, together with the pressurized gas. A non-flammable and non-toxic gas such as carbon dioxide or a chlorofluorocarbon may be employed as the pressurized gas.

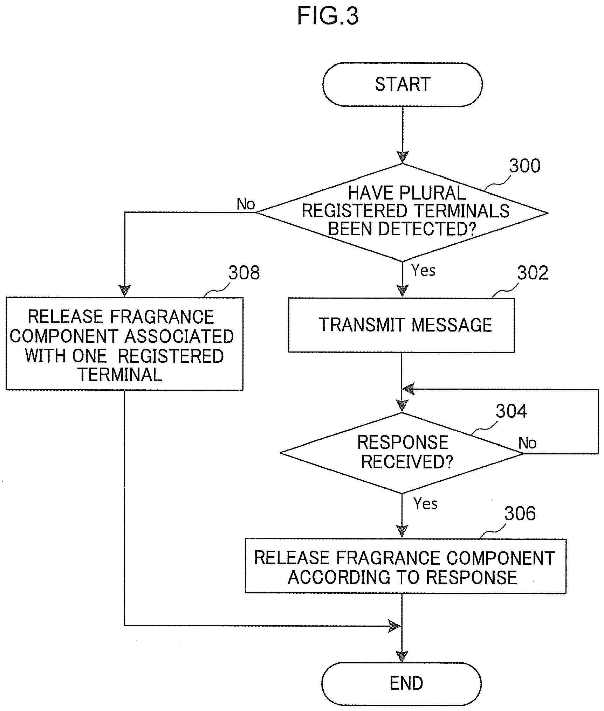

Fig. 3 is a flowchart illustrating an example of processing of the vehicle fragrance dispenser 100 according to the present exemplary embodiment. The processing illustrated in Fig. 3 is started when the transceiver device 60 of the vehicle 10 receives a signal including individual identification information of one of the mobile data terminals 70, 72. In the present exemplary embodiment, the fragrance controller 20 stores individual identification information of the mobile data terminals 70, 72 held by occupants, i.e. users, as registered terminal identification information. The processing illustrated in Fig. 3 is started when individual identification information included in the signals received by the transceiver device 60 matches the stored individual identification information.

At step 300, determination is made as to whether or not plural registered terminals have been detected. The sequence transitions to step 302 when plural registered terminals have been detected, and the sequence transitions to step 308 when a single registered terminal has been detected.

At step 302, a message to select which fragrance to release inside the vehicle is transmitted to the mobile data terminals 70, 72. The message is, for example, a message to prompt selection of any of the perfumes filling the cartridges 34A, 34B, 34C illustrated in Fig. 2.

At step 304, determination is made as to whether or not a response has been received from either of the mobile data terminals 70, 72. When a response has been received, a fragrance component according to the response is released inside the vehicle at step 306, and the processing is ended. Note that when plural responses have been received, the fragrance component according to the response first received, for example, is released inside the vehicle.

When a single registered terminal is detected at step 300, a fragrance component associated with the registered terminal that was detected is released inside the vehicle at step 308, and the processing is ended.

As described above, in the present exemplary embodiment an occupant is identified based on the individual identification information transmitted by the mobile data terminals 70, 72. Accordingly, the present exemplary embodiment may release a fragrance associated with the occupant inside the vehicle. Moreover, the theft of the vehicle 10 may be prevented by releasing tear gas inside the vehicle when the immobilizer 64 has detected an illegitimate engine start attempt.

### [Second Exemplary Embodiment]

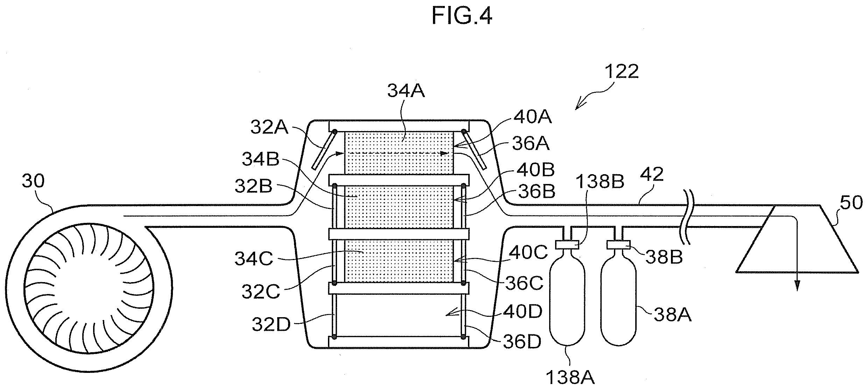

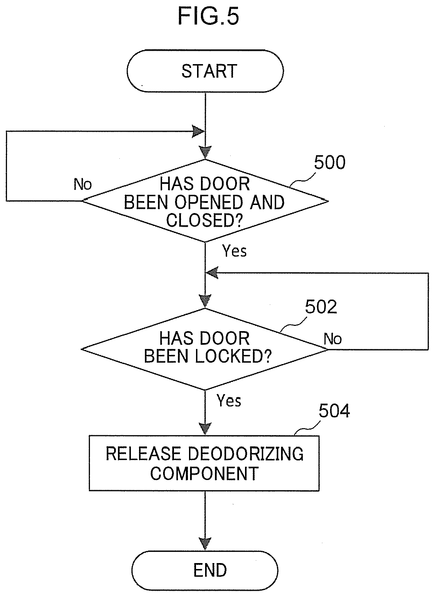

Next, explanation follows regarding a vehicle fragrance dispenser according to a second exemplary embodiment, with reference to Fig. 4 and Fig. 5. In the present exemplary embodiment, after one fragrance component has been released inside the vehicle, a deodorizer is released inside the vehicle before releasing another fragrance component inside the vehicle, thereby deactivating the fragrance component that was released previously.

Fig. 4 is a schematic diagram illustrating an example of a fragrance generator 122 according to the present exemplary embodiment. The vehicle fragrance dispenser according to the present exemplary embodiment differs from the first exemplary embodiment in the point that a deodorizer cartridge 138A and a solenoid 138B are provided to the fragrance generator 122 illustrated in Fig. 4. Other configuration is the same as in the first exemplary embodiment, and such other configuration is accordingly allocated the same reference numerals as in the first exemplary embodiment, and detailed explanation thereof is omitted.

The deodorizer cartridge 138A is, for example, filled with a colloidal solution together with a pressurized gas, with the colloidal solution containing cyclodextrin, a carbohydrate having a ring-shaped structure, dispersed in an aqueous solution of a surfactant. A non-flammable and non-toxic gas such as carbon dioxide or a chlorofluorocarbon may be employed as the pressurized gas, similarly to in the tear gas cartridge 38A.

Cyclodextrin molecules trap molecules of the fragrance component inside the ring structure, thereby deactivating the fragrance component. Molecules of the surfactant in which the cyclodextrin is dispersed also bond to the fragrance component molecules, which has a deactivating effect on the fragrance component.

In the present exemplary embodiment, the deodorizer is released inside the vehicle when it has been detected that the vehicle 10 has stopped and the occupant has left the vehicle, thereby deactivating fragrance component remaining inside the vehicle. When an occupant boards the vehicle 10 anew, similarly to in the first exemplary embodiment, the fragrance generator 22 is controlled so as to release a fragrance component associated with the occupant inside the vehicle.

Fig. 5 is a flowchart illustrating an example of deodorization processing of the vehicle fragrance dispenser according to the present exemplary embodiment. The processing illustrated in Fig. 5 is started when an ignition switch of the vehicle 10 is switched from ON to OFF.

At step 500, determination is made as to whether or not a door on the driver's seat side of the vehicle 10 has been opened and closed. The opening and closing of the door is detected by an existing sensor provided in a door opening and closing mechanism of the vehicle 10.

At step 502, whether or not the doors of the vehicle 10 have been locked is detected. The locking of the doors is detected using an existing sensor provided in a locking mechanism of the vehicle 10.

When locking of the door has been detected at step 502, since this may be determined as the occupant having left the vehicle, the solenoid 138B is opened at step 504 for a predetermined duration, a deodorizing component is released inside the vehicle, and the processing is ended. The predetermined duration differs depending on the size of the interior of the vehicle 10, the type of fragrance component released, and the strength of the fragrance, and so is determined specifically by testing with actual vehicles and actual devices.

As described above, the vehicle fragrance dispenser according to the present exemplary embodiment deactivates fragrance component remaining inside the vehicle by releasing a deodorizer inside the vehicle after the occupant has left the vehicle. Accordingly, the present exemplary embodiment may enable subsequent effective dispersal of a different fragrance component inside the vehicle.

Note that in the configuration in the scope of the patent claims, the fragrance generation section corresponds to the fragrance generator 22, the transceiver section corresponds to the transceiver device 60, and the controller corresponds to the fragrance controller 20.

## Claims

1. A vehicle fragrance dispenser (100) comprising:
a fragrance generation section (22) configured to perfume air within a vehicle (10) using one of a plurality of different perfumes;
a transceiver section (60) configured to communicate with a mobile data terminal (70, 72) of an occupant; and
a controller (20) configured to associate and register any one out of the plurality of perfumes with individual identification information of the mobile data terminal (70, 72) of the occupant, and, in a case in which the transceiver section (60) has received individual identification information of the mobile data terminal (70, 72) of the occupant, the controller (20) controls the fragrance generation section (22) to perfume the air within the vehicle (10) using a perfume associated with the received individual identification information.

2. The vehicle fragrance dispenser (100) of claim 1, wherein, in a case in which the transceiver section (60) has received a plurality of individual identification information associated with the perfumes, the controller (20) transmits a message to mobile data terminals (70, 72) matching the received individual identification information, and controls the fragrance generation section (22) to perfume the air within the vehicle (10) using a perfume indicated by a response to the message.

3. The vehicle fragrance dispenser (100) of either claim 1 or claim 2, wherein:
the fragrance generation section (22) is configured to release a deodorizing component inside the vehicle (10) to deactivate a fragrance component remaining inside the vehicle (10); and
the controller (20) controls the fragrance generation section (22) to release the deodorizing component inside the vehicle (10) in a case in which the occupant has left the vehicle (10) has been detected.

4. The vehicle fragrance dispenser (100) of any one of claim 1 to claim 3, wherein:
the fragrance generation section (22) is configured to release a tear gas component inside the vehicle (10); and
the controller (20) controls the fragrance generation section (22) to release the tear gas component inside the vehicle in a case in which an illegitimate engine start has been detected by an immobilizer (64) of the vehicle (10).
